# EUROPÄISCHE PATENTSCHRIFT

(11) **EP 1 663 332 B1**
(45) Veröffentlichungstag und Bekanntmachung des Hinweises auf die Patenterteilung: **26.01.2011**
(21) Anmeldenummer: 03732484.5
(22) Anmeldetag: 27.05.2003
(51) Int. Cl.: A61L 27/36, A61F 2/24, C12M 3/00

(54) **BIOREAKTOR ZUM HERSTELLEN EINER GEWEBEPROTHESE, INSBESONDERE HERZKLAPPE**
BIOREACTOR FOR PRODUCING A TISSUE PROSTHESIS, PARTICULARLY A HEART VALVE
BIOREACTEUR POUR PRODUIRE UNE PROTHESE TISSULAIRE

(30) Priorität: 16.05.2003 DE 10322024
(43) Veröffentlichungstag der Anmeldung: 07.06.2006
(73) Patentinhaber: Universität Zürich, 8006 Zürich (CH)
(72) Erfinder: COVELLI, Bruno, CH-8005 Zürich (CH); MÜLLI, Rolf, CH-8005 Zürich (CH)
(74) Vertreter: Bremi, Tobias Hans
(86) Internationale Anmeldenummer: PCT/EP2003/005582
(87) Internationale Veröffentlichungsnummer: WO 2004/101012

(56) Entgegenhaltungen:
- DE-A- 10 053 014
- DE-A- 19 919 625
- US-A- 5 899 937
- SODIAN R ET AL: "NEW PULSATILE BIORECTOR FOR FABRICATION OF TISSUE-ENGINEERED PATCHES" JOURNAL OF BIOMEDICAL MATERIALS RESEARCH, WILEY, NEW YORK, NY, US, Bd. 58, Nr. 4, 2001, Seiten 401-405, XP001037742 ISSN: 0021-9304

## Beschreibung

Die vorliegende Erfindung betrifft einen Bioreaktor zum Herstellen einer Gewebeprothese, insbesondere Herzklappe, mit einer Reaktorkammer zum Aufnehmen eines fluiden Nährmediums und eines Prothesenträgers, einer Einrichtung zum Anordnen des Prothesenträgers im Bereich der Reaktorkammer und einer Antriebseinrichtung zum Erzeugen einer Pulsationsströmung des Nährmediums in der Reaktorkammer. Des Weiteren bezieht sich die Erfindung auf ein Verfahren zum Betreiben eines Bioreaktors.

Ein solcher Bioreaktor sowie das zugehörige Verfahren sind aus der DE 19919625 A1 bekannt. Insbesondere die Grundlagen zur In-Vitro-Herstellung einer homologen Herzklappe sind in dieser Druckschrift eingehend erläutert. Bevorzugt wird ein Prothesenträger aus einem biologisch abbaubaren Trägermaterial mit körpereigenen Zellen, vorzugsweise mit Fibroplasten, Myofibroplasten und/oder Endothelzellen besiedelt. Der Prothesenträger gibt dabei vor, welche Gefäße hieraus gezüchtet werden sollen. Insbesondere eignet sich diese Technik zur Züchtung von Herzklappen. Bevor jedoch ein besiedelter Prothesenträger als Ersatzteil in einem Körper eingepflanzt werden kann, müssen die auf dem Prothesenträger angesiedelten Zellen eine stabile Matrix ausbilden, die mechanisch belastbar ist und dementsprechend eine bindegewebsartige Grundstruktur ausbilden muss. Solche stabilen Zellverbände für Gefäße und Herzklappen können in den oben erwähnten Bioreaktoren erzeugt werden. In diesen Bioreaktoren umströmt eine Nährflüssigkeit für die Zellen den besiedelten Prothesenträger pulsförmig. Die Scherkräfte der Strömungspulse auf den Zelloberflächen provozieren die Zellen, einen stabilen, mechanischen belastbaren Zellverband auf dem Träger auszubilden. Die Struktur dieses künstlich gewachsenen Bindegewebes enthält neben den aufgebrachten Fibroplasten und Endothelzellen auch die wesentlichen Bestandteile einer normalen Matrix wie Collagen, Elastin und Glycosaminoglykane.

Bei den bekannten Bioreaktoren werden Pulsfrequenzen zum Erzeugen der Pulsationsströmung ähnlich der Herzfrequenz zwischen 30 - 150 Pulse/min. verwendet. Diese Bioreaktoren verwenden eine elastische Wand oder Membran, die bevorzugt den Boden der Reaktorkammer bildet. Mittels eines mechanischen Antriebs oder über pneumatische Druckänderungen wird der elastischen Wand bzw. der Membran eine pulsartige Bewegung aufgeprägt, wodurch das Nährmedium in der Reaktorkammer dementsprechend das künstliche Gewebe umströmt. Der Aufbau und das Prinzip eines solchen Antriebs sind in der oben erwähnten Druckschrift eingehend beschrieben.

Zur Steigerung der Pulsfrequenz kann somit auch die Fördermenge erhöht und dadurch die Scherkräfte auf das künstliche Gewebe beeinflusst werden. Dieses Verfahrensprinzip hat sich bewehrt und wird heute schon erfolgreich eingesetzt. Dennoch ist man bestrebt, auch auf diesem Gebiet Verbesserungen, insbesondere hinsichtlich einer günstigeren Ansiedlung von Zellen, bei der Erzeugung einer stabilen Matrix herbeizuführen.

Die DE 10053014 beschreibt ein Verfahren zur *in vitro* Herstellung von vitalem, biologischem Ersatzgewebe unter Verwendung von Zellkulturen von einem Trägermaterial. Das Zellgewebe-Konstrukt wird dann einer mechanischen Beanspruchung ausgesetzt.

Sodian et al. (2001) J. Biomed. Mater. Res. 58, 401-405 beschreibt denselben Bioreaktor wie in der DE 10053014. Die Gewebeprothese wird sowohl einem pulsatilen Fluß als auch eine mechanische Beanspruchung durch abwechselnde Dehnung des Zellgewebe-Konstrukts ausgesetzt.

US 5899937 beschreibt einen pulsatilen Fluß System zur Entwicklung von Herzklappen, in denen die Prothese sowohl einen pulastilen Fluß als auch unterschiedlichen Drücken ausgesetzt wird.

Es ist daher die Aufgabe der vorliegenden Erfindung, einen Bioreaktor und ein Verfahren zum Betreiben des Bioreaktors bereitzustellen, die eine Verbesserung bei der Züchtung einer Gewebeprothese bewirken.

Diese Aufgabe wird bei einem Bioreaktor der eingangs genannten Art dadurch gelöst, dass eine Erregereinrichtung zum Erzeugen einer der Pulsationsströmung des Nährmediums überlagerten Frequenzanregung des Nährmediums und/oder des Prothesenträgers vorgesehen ist, und dass die Erregeseinrichtung einen elektromagnetischen Frequenzerzenger umfasst.

Die Erfindung baut demnach auf dem bisher bekannten Bioreaktor auf, der über eine oder mehrere elastische Membranen eine herzähnliche Pulsfrequenz auf das Nährmedium übertragen kann. Zusätzlich wird dem Nährmedium und/oder dem Prothesenträger eine überlagerte Frequenz aufgeprägt. Diese Frequenzanregung soll die Zellen (insbesondere undifferenzierte Zellen, wie Progenitor und Stammzellen) auf dem Prothesenträger zu vermehrtem Wachstum, Differenzierung und zu einer stabilen Matrixbildung anregen. Die Erfinder haben nämlich bei einer strömungsmechanischen Analyse der Vorgänge zwischen Nährflüssigkeit und der Zellmatrix erkannt, dass die hochturbulenten Strömungsimpulse die Grenzschicht der Nährflüssigkeit auf der Zellmatrix aufreißen und die Zellen einerseits mechanisch auf anderen Frequenzen als der Frequenz des pulsierenden Nährmediums beanspruchen und andererseits einen besseren Stoffaustausch bewirken. Dem pulsierenden Nährmedium wird daher zusätzlich eine andere Frequenz als die Pulsfrequenz aufgeprägt, um so die oben erwähnten Effekte zu verstärken. Abschätzungen zeigen, dass bei optimaler Wahl dieser anderen Frequenzen auch Resonanzphänomene im Zellverband auftreten können, die die Zellen zu besonderer Aktivität anregen. Soweit in dieser Anmeldung der Begriff "Prothesenträger" verwendet wird, ist mit zu berücksichtigen, dass dieser bereits von Zellen besiedelt sein kann und dass in einigen Ausführungsbeispielen sich der Prothesenträger bestimmungsgemäß abbaut und die hier beschriebenen Vorgänge bei einem fortgeschrittenen Stadium der Anzüchtung auch unmittelbar an der Gewebeprothese bzw. einem Vorstadium von dieser ablaufen.

Gemäß einer vorteilhaften Ausgestaltungsvariante des Bioreaktors ist es vorgesehen, dass die Erregereinrichtung hinsichtlich ihrer Erregerfrequenz einstellbar ausgestaltet ist. Die Erregerfrequenz könnte auch hinsichtlich ihrer Amplitude einstellbar ausgestaltet sein. In Abhängigkeit verschiedenster Parameter kann durch die Einstellung der Erregerfrequenz der optimale Betriebspunkt für die Anzüchtung der Zellen eingestellt werden. Wie oben bereits erwähnt, können Resonanzphänomene die Zellen zu besonderer Aktivität anregen. Die Einstellbarkeit hilft bei der optimalen Erzeugung solcher Resonanzphänomene.

Wie oben ausgeführt umfasst die Erregereinrichtung einen elektromagnetischen Frequenzerzeuger. Versuche haben gezeigt, dass insbesondere Frequenzen Verwendung finden sollten, die höher sind als die Frequenz der Pulsationsströmung des Nährmediums. Solche Frequenzen sind besonders gut mit elektromagnetischen Frequenzerzeugern erzielbar. Des Weiteren lässt sich ein elektromagnetischer Frequenzerzeuger mit kostengünstigen Mitteln präzise auf die gewünschte Frequenz einstellen.

Darüber hinaus kann die Erregereinrichtung mindestens eine in der Reaktorkammer angeordnete Schallsonde umfassen. Dies bietet die Möglichkeit, die Zellen in einem akustischen Schallfeld auf dem Trägermaterial zu vermehrtem Wachstum, Differenzierung und zu einer stabileren Matrixbildung anzuregen. Der Schalleintrag erfolgt bevorzugt in das Nährmedium und wird somit indirekt auf den Prothesenträger übertragen.

Um die Funktionssicherheit der Erregereinrichtung zu erhöhen, kann diese mindestens eine Antriebseinheit aufweisen, die außerhalb der Reaktorkammer angeordnet ist. Somit ist die Antriebseinheit nicht dem fluiden Nährmedium ausgesetzt. Aufgrund der Tatsache, dass der Bioreaktor bevorzugt in einem Inkubator angeordnet wird, kann die mindestens eine Antriebseinheit auch außerhalb des Inkubators angeordnet werden, so dass keine Erwärmungsprobleme der Antriebseinheit auftreten.

Eine günstige Ausgestaltung der mindestens einen Antriebseinheit und der mindestens einen Schallsonde sieht vor, dass eine Membran als Kopplungsglied zwischen disen beiden Einheiten angeordnet ist, wobei die Membran einen Fensterbereich der Reaktorkammer verschließt. Hierdurch entsteht eine fluiddichte Entkopplung zwischen Schallsonde und Antriebseinheit.

Gemäß einer weiteren Ausführungsform kann die Erregereinrichtung eine Dehn-und/oder Stauchbewegung auf den Prothesenträger übertragend ausgestaltet sein. In diesem Zusammenhang könnte auch die Einrichtung zum Anordnen des Prothesenträgers entsprechend zur Ausführung dieser Bewegungsformen ausgebildet werden. Diese der Pulsationsströmung des Nährmediums überlagerten Dehn- und/oder Stauchbewegungen verstärken die oben bereits erwähnten Effekte, die zu einer besseren Anzüchtung beitragen.

Das Verfahren zum Betreiben eines Bioreaktors zum Herstellen einer Gewebeprothese, insbesondere Herzklappe, zeichnet sich durch folgende Schritte aus:
Anordnen eines Prothesenträgers in einer Reaktorkammer,
Erzeugen einer Pulsationsströmung eines fluiden, in der Reaktorkammer befindlichen Nährmediums,
Erzeugen einer, der Pulsationsströmung überlagerten Frequenzanregung des Nährmediums und/oder Prothesenträgers,
wobei die Frequenz der Pulsationsströmung wiedriger ist, als die Frequenz der überlagerten Frequenzanregung.

Es ist von Vorteil, wenn die Frequenz der Pulsationsströmung niedriger ist, als die Frequenz der überlagerten Frequenzanregung. Insbesondere höhere Frequenzen der überlagerten Frequenzanregung sorgen für das Aufreißen der Grenzschicht der Nährflüssigkeit auf der Zellmatrix, wodurch die Zellen mechanisch auf höheren Frequenzen beansprucht werden. Darüber hinaus bewirkt dies auch einen besseren Stoffaustausch.

Günstig haben sich hierbei Frequenzen erwiesen, die bei der Pulsationsströmung zwischen 0 und 300 Pulsen/min. und bei der überlagerten Frequenzanregung zwischen 0 und 30 kHz liegen. Die überlagerte Frequenzanregung kann hierbei mittels elektrischen, elektromechanischen oder mechanischen Kräften erfolgen.

Als besonders einfache Verfahrensvariante hat sich erwiesen, wenn als überlagerte Frequenzanregung Schallwellen in die Nährflüssigkeit eingebracht werden. Die Einleitung der Schallwellen kann derart erfolgen, dass sich die Zellen in einem akustischen Schallfeld befinden.

Eine weitere Möglichkeit der überlagerten Frequenzanregung besteht darin, dass der Prothesenträger wiederholt gedehnt und/oder gestreckt wird. Diese Dehnung und/oder Streckung kann auch zusammen mit der indirekten Anregung über das Nährmedium erfolgen und so den Effekt noch verstärken.

Als weiterer Vorteil hat sich erwiesen, wenn ein variabler Druckgradient über dem Prothesenträger bzw. der Gewebeprothese erreicht wird. Hierzu ist bei einer weiteren Verfahrensvariante vorgesehen, dass die Anordnung des Prothesenträgers in der Reaktorkammer und/oder die Art und Weise der Erzeugung der Pulsationsströmung in dem fluiden Nährmedium und/oder die Art und Weise der Erzeugung der überlagerten Frequenzanregung derart erfolgt, dass sich dieser Druckgradient entlang der Gewebewand einstellt. Auch dieser Effekt sorgt für eine größere Anregung der Zellen und somit zu einer stabileren Matrixbildung.

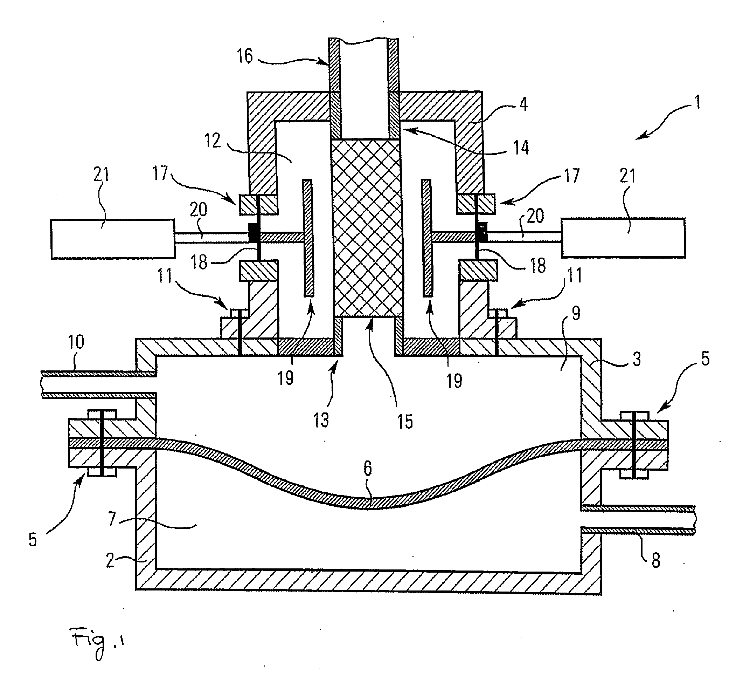

Im Folgenden wird anhand einer Zeichnung eine Ausführungsform eines erfindungsgemäßen Bioreaktors näher beschrieben. Die einzige Figur zeigt eine schematische Darstellung eines Bioreaktors im Vollschnitt.

Die Abmessungen des Bioreaktors sind abhängig von der Größe der herzustellenden Gewebeprothese, das später in den Wirtskörper der Zellen implantiert werden soll.

Der in der Figur dargestellte Bioreaktor 1 weist ein Gehäuse auf, das im Wesentlichen aus drei Bestandteilen aufgebaut ist. Bei den Gehäusebestandteilen handelt es sich um eine untere Gehäuseschale 2, einer darauf angeordneten mittleren Gehäuseschale 3 und einen auf der mittleren Gehäuseschale 3 angeordneten Reaktorkopf 4. Die Gehäuseschale 2 und mittlere Gehäuseschale 3 sind an ihren Randbereichen über Schraubverbindungen 5 unter Zwischenfügung einer elastischen Membran 6 miteinander verbunden. Aufgrund der elastischen Membran 6 ist in der Gehäuseschale 2 eine Pulsationskammer 7 gebildet, die über eine seitlich in die Gehäuseschale 2 mündende Anschlussleitung 8 mit einem Antriebsmedium (Gas oder Flüssigkeit) versorgt wird. Am anderen Ende der Anschlussleitung 8 befindet sich ein Pulsationsantrieb, der hier nicht näher beschreiben wird (siehe z.B. DE 19919625 A1). Oberhalb der elastischen Membran 6 ist in der mittleren Gehäuseschale 3 eine Reaktorvorkammer 9 ausgebildet, die über eine seitlich einmündende Versorgungsleitung 10 mit einem nicht näher dargestellten Vorratsbehälter für Nährflüssigkeit in Verbindung steht.

Der Reaktorkopf 4 ist oben auf die mittlere Gehäuseschale 3 aufgesetzt und mittels Schraubverbindungen 11 mit dieser verbunden. Eine nicht näher dargestellte Dichtung sorgt im Verbindungsbereich für einen sicheren Verschluss. Der Reaktorkopf 4 umgibt die Hauptreaktorkammer 12 des Gehäuses.

Im Übergangsbereich von der Reaktorvorkammer 9 und der Hauptreaktorkammer 12 befindet sich eine untere Trägereinrichtung 13 und im oberen Bereich des Reaktorkopfs 4 befindet sich eine obere Trägereinrichtung 14. Der schematisch dargestellte Prothesenträger 15 ist zwischen der unteren Trägereinrichtung 13 und der oberen Trägereinrichtung 14 gehalten.

Auf der Oberseite des Reaktorkopfes 4 befindet sich eine Abströmleitung 16, über die die Nährflüssigkeit in den Vorratsbehälter abfließen kann, so dass ein im Wesentlichen geschlossener Kreislauf zwischen der Versorgungsleitung 10 und der Abströmleitung 16 entsteht.

Auf beiden Seiten des Reaktorkopfes 4 ist jeweils ein Fenster 17 angeordnet, dessen Öffnung jeweils über eine elastische Membran 18 verschlossen ist. Auf der der Hauptreaktorkammer 12 zugewandten Seite der Membranen 18 befindet sich jeweils eine Antenne oder Sonde 19. Die Sonden 19 erstrecken sich in die Hauptreaktorkammer 12 hinein und erstrecken sich zumindest teilweise im Abstand entlang des Prothesenträgers 15. Auf der der Sonde 19 abgewandten Seite der Membran 18 sind Schallleiter 20 angeordnet, die jeweils mit einem Frequenzgeber verbunden sind. Im vorliegenden Fall erfolgt die Anregung über die Frequenzgeber mittels elektromagnetischer Kräfte. Allerdings könnten auch rein elektrische oder mechanische Kräfte zur Anwendung kommen.

Die untere Trägereinrichtung 13 und obere Trägereinrichtung 14 sind so ausgestaltet, dass sie auch eine Dehn- und Stauchbewegung auf den Prothesenträger 15 ausüben können.

Im Folgenden wird die Wirkungs- und Funktionsweise des oben beschriebenen Bioreaktors 1 näher erläutert.

Bezüglich der Entstehung der Gewebeprothese und der hierfür erforderlichen weiteren Verfahrensschritte wird auf die DE 19919625 A1 verwiesen. Die folgende Beschreibung befasst sich daher hauptsächlich mit der Funktionsweise des Bioreaktors.

In die Pulsationskammer 7 wird ein Druckmedium, im vorliegenden Ausführungsbeispiel eine Flüssigkeit, über die Anschlussleitung eingebracht. Dieses Medium strömt über die Anschlussleitung in und aus der Pulsationskammer 7 heraus, so dass durch die sich dabei hin- und herbewegende elastische Membran 6 Pulsationsfrequenzen zwischen 0 bis 300 Pulsen/min. erzeugt werden können. Die Pulsation der elastischen Membran 6 wird auf die mit Nährflüssigkeit gefüllte Reaktorvorkammer 9 übertragen. Die Pulsform ist in der Regel sinusförmig, es hat sich aber gezeigt, dass Pulsformen in der Art des Herzschlages auf einige kardiovaskuläre künstliche Gewebe eine stabilisierende Wirkung haben. Deshalb kann die nicht dargestellte Antriebseinheit (Pumpe) auch eine nicht sinusförmige Pulsation erzeugen.

Durch diesen Antrieb wird der Nährflüssigkeit die Pulsation des Mediums in der Pulsationskammer 7 aufgeprägt. Durch eine Anordnung von geeigneten Rückschlagventilen in der Versorgungsleitung 10 und der Abströmleitung 16 erfolgt eine gepulste Förderung der Nährflüssigkeit aus der Reaktorvorkammer 9 in die Hauptreaktorkammer 12. Die untere Trägereinrichtung 13 ist so ausgestaltet, dass diese Strömung ohne weiteres möglich ist. Die Nährflüssigkeit durchströmt die Hauptreaktorkammer 12 und strömt dabei entlang dem Prothesenträger 15. Am oberen Ende verlässt überschüssige Nährflüssigkeit die Hauptreaktorkammer 12 über die Abströmleitung 16.

Die Strömung des Nährmediums in der Reaktorvorkammer 9 und der Hauptreaktorkammer 12 kann im Prinzip in zwei Betriebsarten eingestellt werden:
1. Die Versorgungsleitung 10 für die Reaktorvorkammer 9 wird geschlossen und die Abströmleitung 16 aus dem Reaktorkopf 4 ist geöffnet. Dadurch wird die Nährflüssigkeit pulsförmig um den Prothesenträger 15 bzw. das künstliche Gewebe gefördert mit einer Auf- und Abwärtsbewegung.
2. In der Versorgungsleitung 10 und in der Abströmleitung 16 sind die besagten Ventile eingebaut, die eine Rückströmung verhindern. Dadurch wird die Nährflüssigkeit pulsförmig um den Prothesenträger 15 bzw. das künstliche Gewebe gefördert ohne Rückströmung. Die Nährflüssigkeit wird somit von einem nicht dargestellten Vorratsbehälter durch die Versorgungsleitung 10 in den Bioreaktor 1 und von dort über die Abströmleitung 16 in einen Auffangbehälter (respektive den Vorratsbehälter) gefördert.

Auch eine Kombination beider Betriebsarten ist alternierend möglich.

Während die Nährflüssigkeit an dem Prothesenträger 15 bzw. dem künstlichen Gewebe vorbeiströmt, erregen die Frequenzgeber 21 über die Schallleiter 20 die Sonden 19 in der Hauptreaktorkammer 12. Die Frequenz kann im Bereich von 0 bis mehreren kHz eingestellt werden. Im vorliegenden Ausführungsbeispiel werden elektromagnetische Frequenzgeber 21, ähnlich den Antriebseinheiten von akustischen Lautsprechern eingesetzt. Hierdurch wird eine überlagerte Frequenzanregung der Nährflüssigkeit in der Hauptreaktorkammer 12 erzeugt, die die oben beschriebene positive Wirkung für die Anzüchtung von Zellen hat. Die Ausgestaltung der Sonden 19 kann verschieden erfolgen. Prinzipiell besteht auch die Möglichkeit, diese Sonde in Form einer Hülse ringförmig um den Prothesenträger 15 bzw. das künstliche Gewebe anzuordnen.

Die untere Trägereinrichtung 13 und obere Trägereinrichtung 14 sind so ausgestaltet, dass sie die Strömung der Nährflüssigkeit möglichst wenig beeinflussen. Die Geometrie dieser Einrichtungen sind an die Formen des Prothesenträgers 15 bzw. des künstlichen Gewebes angepasst. Vorzugsweise kann als Trägereinrichtung 13 bzw. 14 ein chemisch inertes Kunststoffgerüst eingesetzt werden, das mit dem Prothesenträger 15 bzw. dem künstlichen Gewebe über Klemmen verbunden ist. Durch die Anordnung des Prothesenträgers 15 bzw. des künstlichen Gewebes in der Hauptreaktorkammer 12 und/oder der Art und Weise der Pulsationsströmung und/oder der Art und Weise der übergeordneten Frequenzanregung über die Sonden 19 kann ein Druckgradient über die Wand des Prothesenträgers 15 bzw. des gezüchteten Gewebes zur Nährflüssigkeit erzielt werden.

Die kombinatorische Erregungseinwirkung auf den Prothesenträger 15 bzw. künstliche Gewebe in der Hauptreaktorkammer 12 führt zu vermehrtem Wachstum, Differenzierung und zu einer stabilen Matrixbildung, so dass eine haltbare Gewebeprothese erzeugt wird.

## Patentansprüche

1. Bioreaktor (1) zum Herstellen einer Gewebeprothese, insbesondere Herzklappe, mit einer Reaktorkammer (12) zum Aufnehmen eines fluiden Nährmediums und eines Prothesenträgers (15), einer Einrichtung (13,14) zum Anordnen des Prothesenträgers (15) im Bereich der Reaktorkammer (12) und einer Antriebseinrichtung (6,7) zum Erzeugen einer Pulsationsströmung in der Reaktorkammer (12), **dadurch gekennzeichnet, dass** eine Erregereinrichtung zum Erzeugen einer der Pulsationsströmung des Nährmediums überlagerten Frequenzanregung des Nährmediums und/oder des Prothesenträgers (15) vorgesehen ist, und dass die Erregereinrichtung einen elektromagnetischen Frequenzerzeuger umfasst.

2. Bioreaktor (1) nach Anspruch 1, **dadurch gekennzeichnet, dass** Erregereinrichtung hinsichtlich ihrer Erregerfrequenz einstellbar ausgestaltet ist.

3. Bioreaktor (1) nach Anspruch 1, **dadurch gekennzeichnet, dass** die Erregereinrichtung mindestens eine in der Reaktorkammer (12) angeordnete Schallsonde (19) umfasst.

4. Bioreaktor (1) nach einem der Ansprüche 1 bis 3, **dadurch gekennzeichnet, dass** die Erregereinrichtung mindestens eine Antriebseinheit (21) aufweist, die außerhalb der Reaktorkammer (12) angeordnet ist.

5. Bioreaktor (1) nach Anspruch 4, **dadurch gekennzeichnet, dass** die Reaktorkammer (12) mindestens einen durch eine Membran (18) verschlossenen Fensterbereich (17) aufweist und die Membran (18) als Kopplungsglied zwischen der mindestens einen Antriebseinheit (21) und der mindestens einen Schallsonde (19) ausgestaltet ist.

6. Bioreaktor (1) nach einem der vorangegangenen Ansprüche, **dadurch gekennzeichnet, dass** die Erregereinrichtung eine Dehn- und/oder Stauchbewegung auf den Prothesenträger (15) übertragend ausgestaltet ist.

7. Verfahren zum Betreiben eines Bioreaktors (1) zum Herstellen einer Gewebeprothese, insbesondere Herzklappe, **gekennzeichnet durch**:
Anordnen eines Prothesenträgers (15) in einer Reaktorkammer (12),
Erzeugen einer Pulsationsströmung eines fluiden, in der Reaktorkammer (12) befindlichen Nährmediums,
Erzeugen einer, der Pulsationsströmung überlagerten Frequenzanregung des Nährmediums und/oder des Prothesenträgers (15),
wobei die Frequenz der Pulsationsströmung niedriger ist als die Frequenz der überlagerten Frequenzanregung.

8. Verfahren nach Anspruch 7, **dadurch gekennzeichnet, dass** die Frequenz der Pulsationsströmung zwischen 0 und 300 Hz und die Frequenz der überlagerten Frequenzanregung zwischen 0 und 30 kHz liegt.

9. Verfahren nach einem der Ansprüche 7 oder 8, **dadurch gekennzeichnet, dass** als überlagerte Frequenzanregung Schallwellen in das Nährmedium eingebracht werden.

10. Verfahren nach einem der Ansprüche 7 bis 9, **dadurch gekennzeichnet, dass** als überlagerte Frequenzanregung der Prothesenträger (15) wiederholt gedehnt und/oder gestreckt wird.

11. Verfahren nach einem der Ansprüche 7 bis 10, **dadurch gekennzeichnet, dass** durch die Anordnung des Prothesenträgers (15) in der Reaktorkammer (12) und/oder die Art und Weise des Erzeugens der Pulsationsströmung in dem fluiden Nährmedium und/oder die Art und Weise des Erzeugens der überlagerten Frequenzanregung ein Druckgradient entlang des Prothesenträgers (15) relativ zum fluiden Nährmedium erzeugt wird.

## Claims

1. Bioreactor (1) for the production of a tissue prosthesis, in particular a heart valve, comprising a reactor chamber (12) for receiving a fluid nutrient medium and a prosthesis support (15), a device (13, 14) for placing the prosthesis support (15) in the region of the reactor chamber (12) and a drive device (6, 7) for generating a pulsation flow in the reactor chamber (12), **characterized in that** the bioreactor further comprises an exciter device for generating a frequency excitation of the nutrient medium and/or of the prosthesis support (15), said frequency excitation being superimposed upon the pulsation flow of the nutrient medium, and that the exciter device comprises an electromagnetic frequency generator.

2. Bioreactor (1) according to claim 1, **characterized in that** the excitation device is adjustable to thereby enable the generation of different exciter frequencies.

3. Bioreactor (1) according to claim 1, **characterized in that** the exciter device comprises at least one sound probe (19) located within the reactor chamber (12).

4. Bioreactor (1) according to one of claims 1 to 3, **characterized in that** the exciter device comprises at least one drive unit (21) disposed outside of the reactor chamber (12).

5. Bioreactor (1) according to claim 4, **characterized in that** the reactor chamber (12) comprises at least one window region (17), the window region being closed by a membrane (18), and the membrane (18) being configured as a coupling member between the at least one drive unit (21) and the at least one sound probe (19).

6. Bioreactor (1) according to one of the preceding claims, **characterized in that** the exciter device is configured to induce a lengthening movement and/or a compressive movement to the prosthesis support (15).

7. Method of operating a bioreactor (1) for the production of a tissue prosthesis, in particular a heart valve, the method comprising:
placing a prosthesis support (15) in a reactor chamber (12),
generating a pulsation flow of a fluid nutrient medium contained in said reactor chamber (12),
generating a frequency excitation of the nutrient medium and/or of the prosthesis support (15), said frequency excitation being superimposed upon the pulsation flow, wherein the frequency of the pulsation flow is lower than the frequency of the superimposed frequency excitation.

8. Method according to claim 7, **characterized in that** the frequency of the pulsation flow is between 0 and 300 Hz, and the frequency of the superimposed frequency excitation is between 0 and 30 kHz.

9. Method according to one of claims 7 or 8, **characterized in that** sound waves are introduced into the nutrient medium as superimposed frequency excitation.

10. Method according to one of claims 7 to 9, **characterized in that** as the superimposed frequency excitation the prosthesis support (15) is repeatedly lengthened and/or stretched.

11. Method according to one of claims 7 to 10, **characterized in that** the placement of the prosthesis support (15) in the reactor chamber (12) and/or the manner in which the pulsation flow is created in the fluid nutrient medium, and/or the manner in which the superimposed frequency excitation is generated, serve to generate a pressure gradient along the prosthesis support (15) relative to the fluid nutrient medium.

## Revendications

1. Bioréacteur (1) destiné à fabriquer une prothèse tissulaire, en particulier une valve cardiaque, et qui présente une chambre (12) de réacteur qui reprend un milieu nutritif fluide et un support (15) de prothèse ainsi qu'un dispositif (13, 14) qui permet de placer le support (15) de prothèse dans la chambre (12) du réacteur et qu'un dispositif d'entraînement (6, 7) qui forme un écoulement pulsé dans la chambre (12) du réacteur,
**caractérisé en ce que**
il présente un dispositif d'excitation destiné à former une excitation en fréquence du milieu nutritif et/ou du support (15) de prothèse qui se superpose à l'écoulement pulsé de fluide nutritif et
**en ce que** le dispositif d'excitation comprend un générateur électromagnétique de fréquences.

2. Bioréacteur (1) selon la revendication 1, **caractérisé en ce que** le dispositif d'excitation est configuré de manière à permettre un ajustement de sa fréquence d'excitation.

3. Bioréacteur (1) selon la revendication 1, **caractérisé en ce que** le dispositif d'excitation comprend au moins une sonde acoustique (19) disposée dans la chambre (12) du réacteur.

4. Bioréacteur (1) selon l'une des revendications 1 à 3, **caractérisé en ce que** le dispositif d'excitation présente au moins une unité d'entraînement (21) disposée à l'extérieur de la chambre (12) du réacteur.

5. Bioréacteur (1) selon la revendication 4, **caractérisé en ce que** la chambre (12) du réacteur présente au moins une fenêtre (17) fermée par une membrane (18) et **en ce que** la membrane (18) est configurée comme organe de couplage entre la ou les unités d'entraînement (21) et la ou les sondes acoustiques (19).

6. Bioréacteur (1) selon l'une des revendications précédentes, **caractérisé en ce que** le dispositif d'excitation est configuré de manière à transmettre un déplacement d'allongement et/ou d'écrasement au support (15) de prothèse.

7. Procédé de gestion d'un bioréacteur (1) destiné à fabriquer une prothèse tissulaire, en particulier une valve cardiaque, **caractérisé par** les étapes qui consistent à :
placer un support (15) de prothèse dans une chambre (12) d'un réacteur,
former un écoulement pulsé d'un milieu nutritif fluide présent dans la chambre (12) du réacteur,
former une excitation en fréquence du milieu nutritif et/ou du support (15) de prothèse qui se superpose à l'écoulement pulsé,
la fréquence de l'écoulement pulsé étant plus basse que la fréquence de l'excitation en fréquence qui y est superposée.

8. Procédé selon la revendication 7, **caractérisé en ce que** la fréquence de l'écoulement pulsé est comprise entre 0 et 300 Hz et la fréquence de l'excitation en fréquence superposée est comprise entre 0 et 30 kHz.

9. Procédé selon l'une des revendications 7 ou 8, **caractérisé en ce que** comme excitation à fréquence superposée, on applique des ondes acoustiques dans le milieu nutritif.

10. Procédé selon l'une des revendications 7 à 9, **caractérisé en ce que** comme excitation superposée en fréquence, le support de prothèse (15) est allongé et/ou étiré de manière répétée.

11. Procédé selon l'une des revendications 7 à 10, **caractérisé en ce qu'**un gradient de pression est formé le long du support de prothèse (15) par rapport au milieu nutritif fluide par le placement du support (15) de prothèse dans la chambre (12) du réacteur et/ou par le type de formation de l'écoulement pulsé dans le milieu nutritif fluide et/ou par le type de formation de l'excitation en fréquence qui y est superposée.
